# EUROPEAN PATENT APPLICATION

(11) **EP 4 407 075 A1**
(43) Date of publication of application: **31.07.2024**
(21) Application number: 22873005.7
(22) Date of filing: 22.09.2022
(51) Int. Cl.: C25F 3/16, A61M 5/32

(54) **PRODUCTION METHOD FOR CONICAL HOLLOW NEEDLE**

(30) Priority: 24.09.2021 JP 2021155204
(71) Applicant: Nipro Corporation, Settsu-shi, Osaka 566-8510 (JP)
(72) Inventor: KIMURA, Daisuke, Osaka-shi, Osaka 531-8510 (JP); EIZUMI, Yutaka, Tatebayashi-shi, Gunma 374-8518 (JP); KUROKAWA, Kenji, Tatebayashi-shi, Gunma 374-8518 (JP); KANEKO, Masato, Tatebayashi-shi, Gunma 374-8518 (JP)
(74) Representative: Slingsby Partners LLP
(86) International application number: PCT/JP2022/035457
(87) International publication number: WO 2023/048254

(57) **Abstract**

Provided is a novel manufacturing method for a conical hollow needle that makes it possible to efficiently form a conical part that has a nearly perfectly circular outer circumferential surface shape. The present invention is a method of manufacturing a conical hollow needle 10 that has a conical part 24 that has a tapered outer circumferential surface 20. The manufacturing method involves immersing a hollow needle 10' having a straight shape in an electrolyte solution 30 to conically electropolish an outer circumferential surface 24'. From a start to an end of the electropolishing, the hollow needle 10', which serves as an anode, is sandwiched between a pair of cathodes 34, 34 at a prescribed distance therefrom in a radial direction, and the hollow needle 10' is rotated around a needle axis thereof to change which faces of the hollow needle 10' are opposite to the cathodes 34, 34.

## Description

### TECHNICAL FIELD

This invention relates to a method of manufacturing a conical hollow needle having a conical part with a tapered outer circumferential surface.

### BACKGROUND ART

Conventionally, hollow needles with approximately constant inner and outer diameters have been used for injection needles and the like. U.S. Patent No. 5,002,535 (Patent Document 1) discloses a conical hollow needle having a tapered outer circumferential surface whose outer diameter at the distal end part is smaller than that at the proximal end part.

### BACKGROUND ART DOCUMENT

### PATENT DOCUMENT

Patent Document 1: U.S. Patent No. 5,002,535

### SUMMARY OF THE INVENTION

### PROBLEM THE INVENTION ATTEMPTS TO SOLVE

Incidentally, the tapered outer circumferential surface of a conical hollow needle may be formed by chemical polishing using a metal-soluble bath such as acid, other than by mechanical polishing. For example, it is conceivable to make the outer circumferential surface of a conical hollow needle tapered by chemically polishing a hollow needle having a straight shape, which is an original form of a conical hollow needle, while controlling the region immersed in a metal-soluble bath and hence the amount of dissolution of the outer circumferential surface in the direction of the needle length.

Meanwhile, in such chemical polishing, in order to improve polishing efficiency and enable industrial production, it is desirable to conduct electrochemical polishing (electropolishing) by placing an electrode plate in the bath and passing an electric current between the electrode plate and the hollow needle.

However, the present inventors' studies have revealed that in the conventional electropolishing process, in which merely one electrode plate is arranged opposite to a hollow needle, the outer circumferential surface shape of the electropolished hollow needle may become a distorted circular shape. That is, it became clear that in the conventional electrochemical polishing using a simple electrode plate, the outer circumferential surface shape of the conical portion is distorted, which may increase the patient's pain during puncture, adversely affect the strength characteristics of the hollow needle, and the like.

It is therefore one object of the present invention to provide a novel method of manufacturing a conical hollow needle which is able to efficiently form a conical part with an outer circumferential surface shape close to a perfect circle.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a method of manufacturing a conical hollow needle including a conical part whose outer circumferential surface is tapered, comprising immersing a hollow needle having a straight shape in an electrolytic solution and performing electropolishing to make an outer circumferential surface of the hollow needle into a conical shape, wherein from a start to an end of the electropolishing, a pair of cathodes are arranged such that the cathodes sandwich the hollow needle serving as an anode at a prescribed distance in a radial direction, and the hollow needle is rotated around a needle axis to change which faces of the hollow needle are opposite to the cathodes.

According to the method of manufacturing a conical hollow needle following this preferred embodiment, the hollow needle is rotated during the step of electropolishing on the hollow needle, thereby changing which faces of the hollow needle are opposite to the cathodes. This will minimize variations in electrolysis efficiency caused by differences in the embodiments of opposition, such as the distance and orientation of the outer circumferential surface of the hollow needle with respect to the cathode. Therefore, the conical part of the outer circumferential surface obtained by electropolishing will be made into an outer circumferential shape closer to a perfect circle, so that pain during puncture is effectively reduced by the decreased diameter. In addition, the cross sections of the inner circumferential surface and the outer circumferential surface of the conical hollow needle are each shaped close to a perfect circle, thereby obtaining a conical hollow needle with excellent strength characteristics.

A second preferred embodiment provides a method of manufacturing a conical hollow needle including a conical part whose outer circumferential surface is tapered, comprising immersing a hollow needle having a straight shape in an electrolytic solution and performing electropolishing to make an outer circumferential surface of the hollow needle into a conical shape, wherein from a start to an end of the electropolishing, at least one cathode is arranged radially opposite to the outer circumferential surface of the hollow needle serving as an anode, the at least one cathode being arranged at not less than three different positions in a circumferential direction of the hollow needle.

According to the method of manufacturing a conical hollow needle following this preferred embodiment, the cathode is arranged at not less than three different positions in the circumferential direction of the hollow needle during the step of electropolishing on the hollow needle. This will minimize variations in electrolysis efficiency caused by differences in the embodiments of opposition, such as the distance and orientation of the outer circumferential surface of the hollow needle with respect to the cathode. Therefore, the conical part of the outer circumferential surface obtained by electropolishing will be made into an outer circumferential shape closer to a perfect circle, and will be decreased in diameter over the entire circumference, so that pain during puncture is effectively reduced by the decreased diameter. In addition, the cross sections of the inner circumferential surface and the outer circumferential surface of the conical hollow needle are each shaped close to a perfect circle, thereby obtaining a conical hollow needle with excellent strength characteristics.

A third preferred embodiment provides the method of manufacturing a conical hollow needle according to the second preferred embodiment, wherein the at least one cathode comprises a plurality of cathodes arranged independently of each other in the circumferential direction of the hollow needle, and from the start to the end of the electropolishing, an arrangement of the plurality of cathodes relative to the hollow needle is changed so that the plurality of cathodes are arranged at different positions in the circumferential direction of the hollow needle.

According to the method of manufacturing a conical hollow needle following this preferred embodiment, the plurality of cathodes are arranged independently in the circumferential direction of the hollow needle. By so doing, the portions of the hollow needle, which are to be efficiently electropolished by being opposite to the cathodes, can be simultaneously obtained in a plurality of directions. In addition, during the electropolishing step, by moving the outer circumferential surface of the hollow needle and the cathodes relative to each other, for example, the cathodes can be positioned at not less than three different positions in the circumferential direction of the hollow needle. This makes it possible to make the conical part closer to a perfect circle with fewer cathodes.

A fourth preferred embodiment provides the method of manufacturing a conical hollow needle according to the second preferred embodiment, wherein the at least one cathode is arranged to surround the hollow needle, and from the start to the end of the electropolishing, the at least one cathode is simultaneously opposite to the hollow needle at not less than three different positions in the circumferential direction.

According to the method of manufacturing a conical hollow needle following this preferred embodiment, for example, the number of cathodes may be not less than three, or the cathode may be curved in the circumferential direction of the hollow needle, or the like so that the cathode is arranged to surround the hollow needle. This makes it possible to make the conical part closer to a perfect circle without requiring rotation of the hollow needle or movement of the cathode.

A fifth preferred embodiment provides the method of manufacturing a conical hollow needle according to any one of the second through fourth preferred embodiments, wherein the at least one cathode extends in a curved manner in the circumferential direction of the hollow needle.

According to the method of manufacturing a conical hollow needle following this preferred embodiment, the region of the hollow needle that is radially opposite to the cathode is increased in the circumferential direction. Thus, the region where efficient electropolishing is possible can be widely obtained in the circumferential direction of the hollow needle.

A sixth preferred embodiment provides the method of manufacturing a conical hollow needle according to any one of the first through fifth preferred embodiments, wherein the conical part with a roundness of 0 to 0.0015 mm is formed by the electropolishing.

According to the method of manufacturing a conical hollow needle following this preferred embodiment, it is possible to obtain a conical hollow needle having a conical part with a cross-sectional shape sufficiently close to a perfect circle on its outer circumferential surface. Therefore, the conical part is made small in diameter over the entire circumference to reduce pain during puncture. Besides, reduction in partial stress concentration in the circumferential direction of the conical hollow needle or the like can be achieved, thereby realizing excellent strength characteristics in the conical hollow needle.

A seventh preferred embodiment provides a method of manufacturing a conical hollow needle including a conical part whose outer circumferential surface is tapered, comprising immersing a hollow needle having a straight shape in an electrolytic solution and performing electropolishing to make an outer circumferential surface of the hollow needle into a conical shape, wherein a plurality of cathodes are arranged apart from and opposite to the outer circumferential surface of the hollow needle serving as an anode, and the electropolishing on the hollow needle by each cathode is adjusted by controlling an energization state between the hollow needle and the plurality of cathodes.

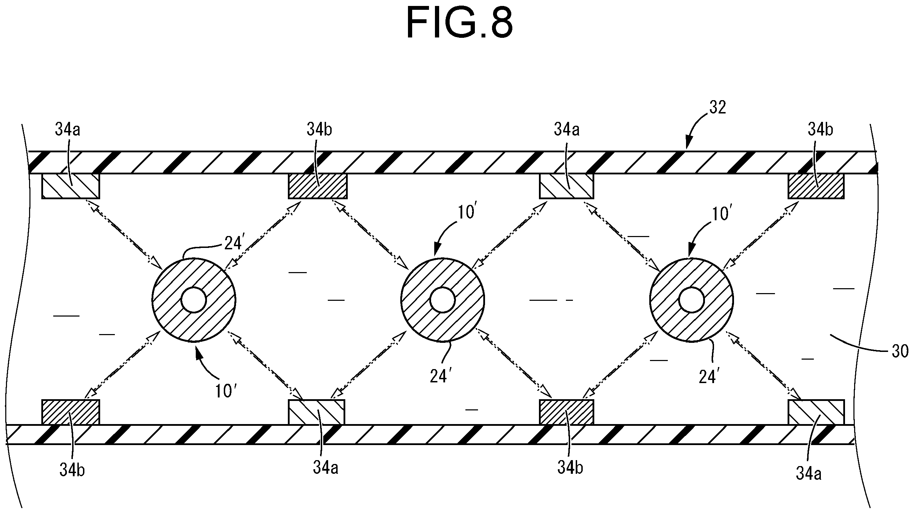

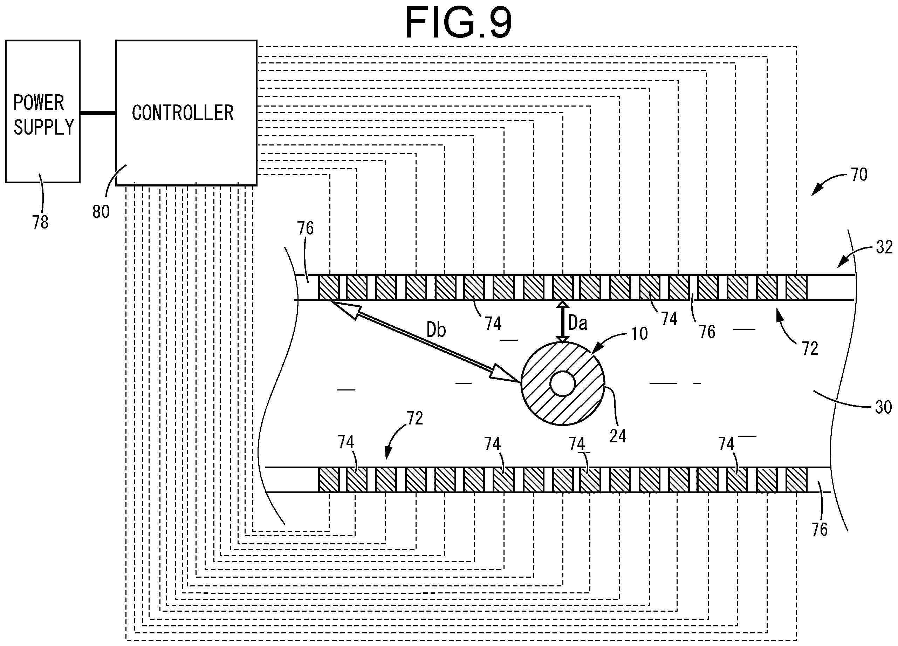

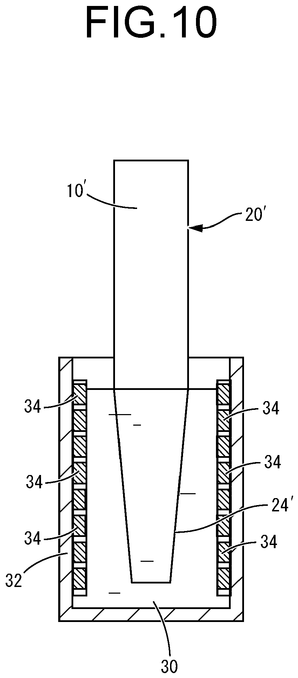

According to the method of manufacturing a conical hollow needle following this preferred embodiment, as illustrated in FIG. 8 of the practical embodiment described later, for example, a plurality of pairs of cathodes are arranged opposite to each other with the hollow needle interposed therebetween in a plurality of diametrical directions. Then, by controlling to alternately switch the energization state between the cathodes and the hollow needle, the region on the hollow needle electropolished by each pair of cathodes is adjusted to switch sequentially in the circumferential direction, thereby improving a roundness of the hollow needle. In addition, as illustrated in FIG. 9 of the practical embodiment described later, for example, a plurality of cathodes are arranged at the positions different from one another in the circumferential direction of the hollow needle. Then, by controlling energization time and/or applied voltage to be mutually adjusted with respect to the cathodes or the like, it is possible to adjust the amount of electropolishing on the hollow needle in the circumferential direction, thereby improving the roundness of the hollow needle. Alternatively, as illustrated in FIG. 10 of the practical embodiment described later, for example, a plurality of cathodes are arranged at the positions different from one another in the length direction of the hollow needle. Then, by controlling energization time and/or applied voltage to be mutually adjusted with respect to the cathodes or the like, it is possible to adjust the amount of electropolishing on the hollow needle in the length direction, thereby achieving a conical shape, adjusting the inclination angle of the conical portion, and the like.

### EFFECT OF THE INVENTION

According to the present invention, it is possible to efficiently form a conical part with an outer circumferential surface shape close to a perfect circle.

### BRIEF DESCRIPTION OF THE DRAWINGS

FIG. 1 is a plan view of a conical hollow needle according to a first practical embodiment of the present invention.
FIG. 2 is a left side view of the conical hollow needle shown in FIG. 1.
FIG. 3 is a cross sectional view taken along line 3-3 of FIG. 1.
FIG. 4A is a view suitable for explaining a manufacturing method of the conical hollow needle shown in FIG. 1, showing a state in which a metal pipe is not immersed in an electrolytic solution during an electropolishing step.
FIG. 4B is a view suitable for explaining the manufacturing method of the conical hollow needle shown in FIG. 1, showing a state in which a distal end portion of the metal pipe is immersed in the electrolytic solution during the electropolishing step.
FIG. 4C is a view suitable for explaining the manufacturing method of the conical hollow needle shown in FIG. 1, showing a state in which an entire electropolishing region of the metal pipe is immersed in the electrolytic solution during the electropolishing step.
FIG. 5A is an enlarged view suitable for explaining the manufacturing method of the conical hollow needle shown in FIG. 1, showing a first electropolishing step.
FIG. 5B is an enlarged view suitable for explaining the manufacturing method of the conical hollow needle shown in FIG. 1, showing a second electropolishing step.
FIG. 6 is a view suitable for explaining a manufacturing method of a conical hollow needle according to a second practical embodiment of the present invention.
FIG. 7 is a view suitable for explaining a manufacturing method of a conical hollow needle according to a third practical embodiment of the present invention.
FIG. 8 is a view suitable for explaining a manufacturing method of a conical hollow needle according to a fourth practical embodiment of the present invention.
FIG. 9 is a view suitable for explaining a manufacturing method of a conical hollow needle according to a fifth practical embodiment of the present invention.
FIG. 10 is a view suitable for explaining a manufacturing method of a conical hollow needle according to a sixth practical embodiment of the present invention.

### EMBODIMENTS FOR CARRYING OUT THE INVENTION

Hereinafter, practical embodiments of the present invention will be described with reference to the drawings.

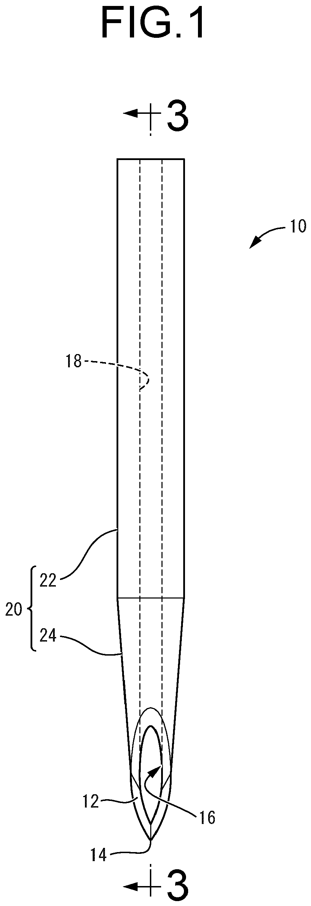

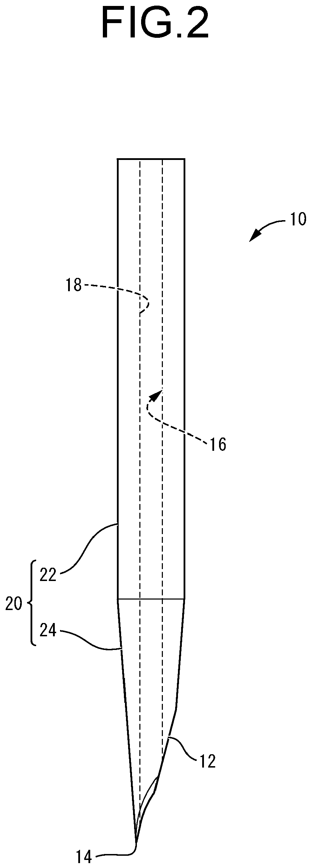

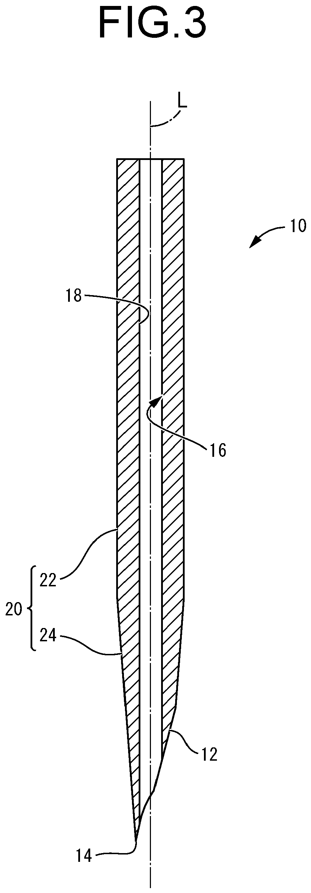

FIGS. 1 to 3 show a conical hollow needle 10 according to a first practical embodiment of the present invention. The conical hollow needle 10 is formed of a metal such as medical stainless steel. The conical hollow needle 10 has a blade surface 12 whose distal end surface inclines with respect to the needle axis L, and has a needle tip 14 at the distal end. The blade surface 12 of this practical embodiment has a different inclination angle with respect to the needle axis L on the distal end side and the proximal end side, with the needle tip 14 being made sharper by having a smaller inclination angle on the distal end side. However, the blade surface 12 may be a single planar shape having a constant inclination angle throughout. The conical hollow needle 10 is penetrated by a lumen 16 in the needle axis direction (the vertical direction in FIG. 1), and has an inner circumferential surface 18 that is a wall surface of the lumen 16. The inner circumferential surface 18 has a cross-sectional shape of an approximately perfect circle and extends straight with an approximately constant cross section in the needle axis direction. That is, the conical hollow needle 10 has an approximately constant inner diameter dimension across the entire length in the needle axis direction.

An outer circumferential surface 20 of the conical hollow needle 10 has a cylinder part 22 at the proximal end portion (the upper portion in FIG. 1) opposite the needle tip 14 that extends straight with an approximately constant cross section, and a tapered conical part 24 at the distal end portion (the lower portion in FIG. 1) on the needle tip 14 side. The conical part 24 inclines at an approximately constant inclination angle with respect to the needle axis L. The inclination angle of the conical part 24 with respect to the needle axis L is suitably not smaller than 0.05 degrees and not greater than 0.35 degrees, and more suitably not smaller than 0.09 degrees and not greater than 0.3 degrees. With this configuration, the distal end part of the conical part 24 can be sufficiently reduced in diameter, while the change of angle between the cylinder part 22 and the conical part 24 can be minimized. Of course, the inclination angle of the conical part 24 with respect to the needle axis L may gradually or stepwise change in the needle axis direction, which is the length direction. In the drawings, the inclination angle of the conical part 24 is exaggerated to make the shape of the conical part 24 easier to understand.

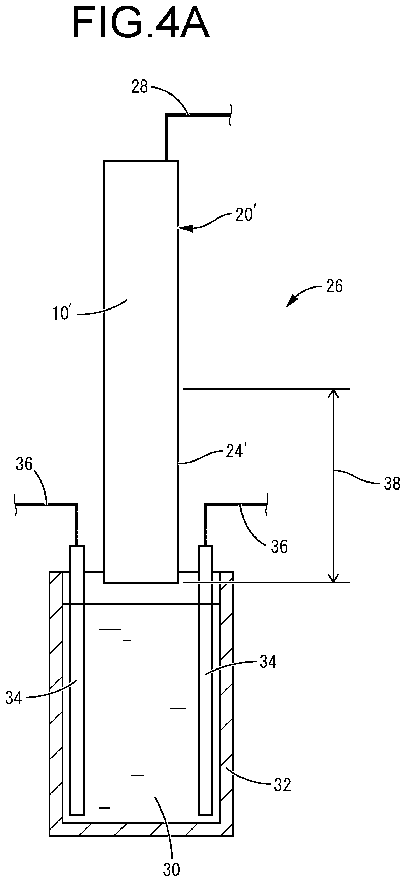

The conical part 24 of the conical hollow needle 10 is formed by performing electropolishing to taper the distal end portion of a metal pipe 10', which is a hollow needle having a straight shape, with an electropolishing device 26 shown in FIGS. 4A to 4C, for example. Therefore, the conical part 24 of the conical hollow needle 10 comprises an electropolished surface formed by electropolishing.

As shown in FIG. 4A, the metal pipe 10', which is an original form of the conical hollow needle 10, has a straight round tubular shape in its initial form before electropolishing, and its inner and outer diameter dimensions are both approximately constant in the axial direction. Besides, anode side wiring 28 is connected to the metal pipe 10'. One end of the anode side wiring 28 is electrically connected to the metal pipe 10', which serves as an anode for electrolysis, and the other end is electrically connected to a power supply device (not shown).

Additionally, an electrolytic cell 32 containing an electrolytic solution 30 is prepared. The electrolytic solution 30 is not particularly limited as long as it can realize electropolishing of the metal pipe 10', but for example, a phosphoric acid aqueous solution prepared by diluting phosphoric acid with water is used.

Further, a pair of electrodes 34, 34, which are opposite to each other and serve as cathodes, are inserted into the electrolytic cell 32. The electrodes 34, 34 are each formed of conductive metal. The electrodes 34, 34 are at least partially immersed in the electrolytic solution 30. The portions of the electrodes 34, 34 immersed in the electrolytic solution 30 have a projected area in the direction of opposition that is not smaller than an electropolishing region 38 (described later) in a side view of the metal pipe 10'. Cathode side wiring 36 is connected to each electrode 34. One end of the cathode side wiring 36 is electrically connected to the electrode 34, which serves as a cathode for electrolysis, and the other end is electrically connected to a power supply device (not shown).

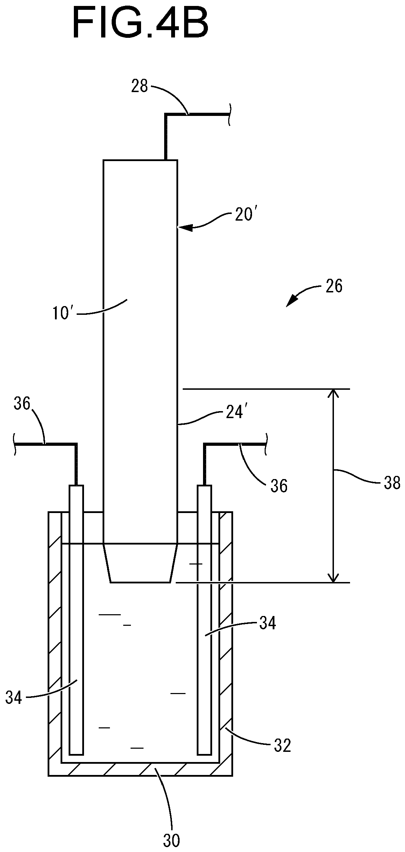

After placing the metal pipe 10' over the opening of the electrolytic cell 32 as shown in FIG. 4A, the metal pipe 10' and the electrolytic cell 32 are brought close to each other, and the distal end portion of the metal pipe 10' is immersed in the electrolytic solution 30 in the electrolytic cell 32 as shown in FIG. 4B. Then, by passing an electric current between the metal pipe 10' immersed in the electrolytic solution 30 and the electrodes 34, 34, with the metal pipe 10' serving as the anode and the electrodes 34, 34 serving as the cathodes, an electrolytic reaction is generated in the metal pipe 10'. By so doing, the portion of the metal pipe 10' immersed in the electrolytic solution 30 is dissolved from the radially outer side to reduce its outer diameter dimension. During the electropolishing step, the two electrodes 34, 34 are arranged such that the electrodes 34, 34 sandwich the metal pipe 10' at a prescribed distance in the radial direction. The two electrodes 34, 34 are arranged independently of each other in the circumferential direction of the metal pipe 10'.

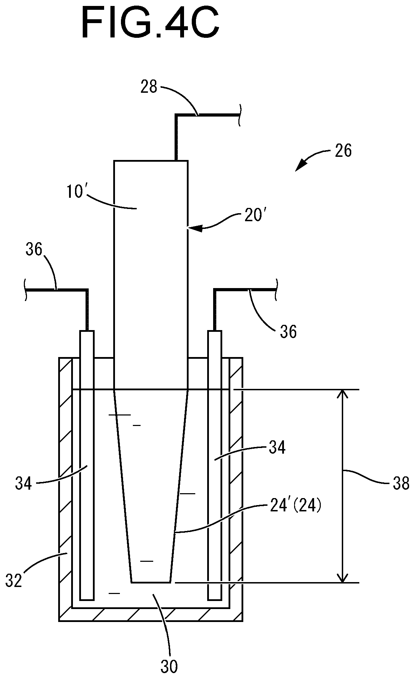

During the electropolishing step, as shown in FIGS. 4B and 4C, the metal pipe 10' and the electrolytic cell 32 are moved relative to each other in the axial direction of the metal pipe 10' (the vertical direction in FIG. 4B) so that the region of the metal pipe 10' immersed in the electrolytic solution 30 is changed. This allows the amount of dissolution of the metal pipe 10' by electropolishing to be different in the axial direction, and the outer diameter dimension of the metal pipe 10' can be changed in the axial direction.

Specifically, for example, by gradually immersing the metal pipe 10' in the electrolytic solution 30 to the more proximal end side, in the electropolishing region 38 of the metal pipe 10' (the length region where the conical part 24 of the conical hollow needle 10 is formed), the time for immersion in the electrolytic solution 30 can be gradually increased from the proximal end to the distal end of the metal pipe 10'. As a result, in the electropolishing region 38 of the metal pipe 10', the distal end side, which is immersed in the electrolytic solution 30 for a relatively long time, has a larger amount of dissolution by electrolysis than the proximal end side, which is immersed in the electrolytic solution 30 for a relatively short time. Therefore, an outer circumferential surface 24' of the electropolishing region 38 of the metal pipe 10' is tapered from the proximal end to the distal end by electropolishing. By so doing, the outer circumferential surface 24' of the electropolishing region 38 of the metal pipe 10' serves as the conical part 24 formed by the electropolished surface after electropolishing. The electropolishing region 38 is the length region in the metal pipe 10' that is immersed in the electrolytic solution 30 during the electropolishing step, and refers to the length region of the metal pipe 10' from the distal end of the metal pipe 10' to the surface of the electrolytic solution 30 in FIG. 4C, which shows the state in which the immersion length of the metal pipe 10' in the electrolytic solution 30 is at its maximum.

In this practical embodiment, during the step from the start to the end of electropolishing, the metal pipe 10' and the electrodes 34, 34 are moved relative to each other to change the direction of opposition of the metal pipe 10' and the electrodes 34, 34. By so doing, during the electropolishing step, the electrodes 34, 34 are arranged at not less than three different positions in the circumferential direction of the metal pipe 10'.

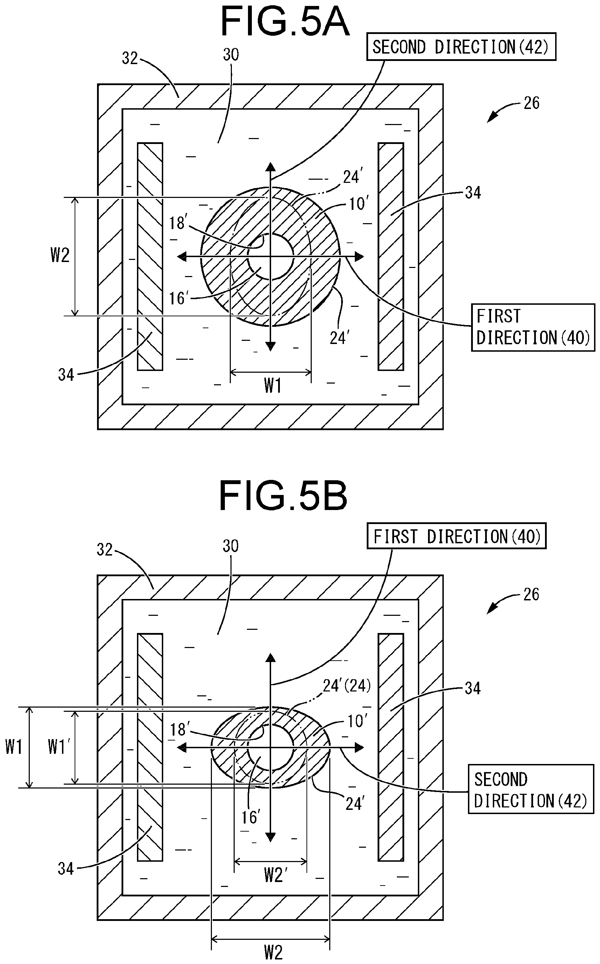

Specifically, for example, first, as shown in FIG. 5A, the first electropolishing step is performed in a first arrangement in which the electrodes 34, 34 are arranged opposite to each other in a first direction 40 of the metal pipe 10'. At this time, the amount of dissolution is greater on the opposite sides in the first direction 40, which is the direction of opposition with respect to the electrodes 34, 34, than on the opposite sides in a second direction 42, which is orthogonal to the first direction 40. Thus, the outer circumferential surface 24' of the electropolishing region 38 in the metal pipe 10' for which the first electropolishing step is completed (the chain double-dashed line in FIG. 5A), has a width dimension W1 in the first direction 40 that is smaller than a width dimension W2 in the second direction 42. In the metal pipe 10' for which the first electropolishing step is completed, the roundness (|W2-W1|) of the outer circumferential surface 24' of the electropolishing region 38 is about 0.0055 mm, for example. The metal pipe 10' for which the first electropolishing step is completed has a wall thickness of the electropolishing region 38 that is thinner in the first direction 40 than in the second direction 42.

After completion of the first electropolishing step, the metal pipe 10' is rotated 90 degrees in the circumferential direction, or the electrodes 34, 34 are moved 90 degrees in the circumferential direction around the metal pipe 10'. By so doing, as shown in FIG. 5B, the electrodes 34, 34 are arranged at the positions opposite to each other in the second direction 42 of the metal pipe 10'. The second electropolishing step is then executed in a second arrangement in which the metal pipe 10' and the electrodes 34, 34 are opposite to one another in the second direction 42. In the second electropolishing step, the amount of dissolution the metal pipe 10' is greater on the opposite sides in the second direction 42 than on the opposite sides in the first direction 40. The metal pipe 10', which has been made thin and small-diameter in the first direction 40 while being thick and large-diameter in the second direction 42 by electropolishing in the first arrangement, is processed by electropolishing in the second arrangement so that the outer circumferential surface 24' of the electropolishing region 38 has a shape closer to a perfect circle, as shown in FIG. 5B with the chain double-dashed line. Specifically, the outer circumferential surface 24' of the electropolishing region 38 of the metal pipe 10' for which the second electropolishing step is completed (the chain double-dashed line in FIG. 5B) has a width dimension W1' in the first direction 40 that is approximately equal to a width dimension W2' in the second direction 42. In the metal pipe 10' for which the second electropolishing step is completed, the wall thickness of the electropolishing region 38 is approximately the same in the first direction 40 and in the second direction 42.

The first electropolishing step and the second electropolishing step can be performed discontinuously, for example, by changing the arrangement of the metal pipe 10' and the electrodes 34, 34 from the first arrangement to the second arrangement with the energization stopped, and then energizing again to resume electropolishing. The first electropolishing step and the second electropolishing step can also be performed continuously by changing the arrangement from the first arrangement to the second arrangement while performing electropolishing. The time required for the first electropolishing step and the time required for the second electropolishing step may be different from each other or may be the same.

In this practical embodiment, during the electropolishing step, by relative movement (relative rotation) of the metal pipe 10' and the electrodes 34, 34, the arrangement of the electrodes 34, which serve as the cathodes, is changed with respect to an outer circumferential surface 20' of the metal pipe 10', which serves as the anode, so that the electrodes 34, 34 are arranged at four different positions in the circumferential direction of the metal pipe 10', thereby changing which faces of the metal pipe 10' are opposite to the electrodes 34. This reduces the differential in the amount of dissolution between the first direction 40 and the second direction 42 of the metal pipe 10', so that the flattening of the outer circumferential surface 24' of the electropolishing region 38 in the metal pipe 10' is prevented, thereby electropolishing the outer circumferential surface 24' of the metal pipe 10' into a shape closer to a perfect circle. That is, the completion of the second electropolishing step makes the roundness of the conical part 24 (|W2'-W1'|) smaller than the value at the completion of the first electropolishing step (e.g. 0.0055 mm). Regarding the metal pipe 10' for which the electropolishing step including the first electropolishing step and the second electropolishing step is completed, the roundness of the conical part 24 (|W2'-W1'|), which is the outer circumferential surface 24' of the electropolishing region 38, is suitably within a range of 0 to 0.0015 mm, which enables the manufacture of needles that are closer to a perfect circle than in the past.

The distal-end outer diameter and the proximal-end outer diameter of the conical part 24 are set such that the differential between the gauge dimensions defined in the ISO9626 is 1, for example, and the distal-end outer diameter is smaller than the proximal-end outer diameter. Specifically, for example, when the proximal end of the conical part 24 has an outer diameter dimension equivalent to 32 gauge in the ISO9626 standard, the distal end of the conical part 24 has an outer diameter dimension equivalent to 33 gauge in the ISO 9626 standard. The gauge dimension of the outer diameter of the metal pipe 10' is approximately the same as the gauge dimension of the proximal-end outer diameter of the conical part 24. However, the outer and inner diameter dimensions of the conical hollow needle 10 and the outer and inner diameter dimensions of the metal pipe 10' do not necessarily have to be set according to the ISO9626 standard (gauge dimensions). Besides, the present invention is also applicable to conical hollow needles thinner than 33 gauge and conical hollow needles thicker than 32 gauge. Although the ISO9626 is a standard for the diameter dimensions of needles (tubes) of a constant outer diameter and does not cover the outer diameter dimensions of tapered tubular surfaces such as the conical part 24 of the conical hollow needle 10, the diameter dimensions at specific positions are described using the gauge dimensions defined in the ISO9626.

Since the inner circumferential surface 18' of the metal pipe 10' is not opposite to the electrodes 34, 34, the change in shape due to electrolysis is minimized compared to the outer circumferential surface 24'. Therefore, the inner circumferential surface 18' of the metal pipe 10' after electropolishing, namely, the inner circumferential surface 18 of the conical hollow needle 10, is maintained in a cross-sectional shape close to a perfect circle. Besides, the inner diameter dimension of the conical hollow needle 10 is approximately the same as that of the metal pipe 10' before electropolishing.

After the metal pipe 10' with the conical part 24 formed by electropolishing is taken out from the electrolytic cell 32, the blade surface 12 and the needle tip 14 are formed by cutting or the like of the distal end to make the conical hollow needle 10. If burrs on the blade surface 12 formed by cutting or the like are a problem, a shot-blasting process or the like may be performed to remove the burrs by applying small balls of glass or metal to the blade surface 12.

According to this practical embodiment, when forming the conical part 24 of the conical hollow needle 10 by electropolishing, the position of the electrode 34 with respect to the metal pipe 10' is set to not less than three positions in the circumferential direction of the metal pipe 10'. This makes it possible to set the portions of the metal pipe 10' that are opposite the electrode 34, in which the electrolysis of the metal pipe 10' is efficiently progressed, to not less than three locations in the circumferential direction. Therefore, compared to the case where the metal pipe 10' and the electrode 34 are opposite to each other in only one or two orientations, the outer circumferential surface 20' of the metal pipe 10' can be entirely electropolished in the circumferential direction to reduce its diameter. As a result, regarding the conical hollow needle 10, the conical part 24 has a transverse cross-sectional shape that is closer to a perfect circle, thereby effectively reducing pain at the time of puncture due to decrease in diameter over the entire circumference. Additionally, the conical hollow needle 10 can achieve excellent strength characteristics due to its circular cross section when external force is applied.

In this practical embodiment, in the electropolishing step, the electrodes 34, 34 are arranged at four locations in the circumferential direction of the metal pipe 10' by rotating the metal pipe 10' and the electrodes 34, 34 relative to each other. Therefore, with fewer than three electrodes 34, 34, the circumferential variation in the electrolytic action can be effectively reduced.

In particular, regarding the two electrodes 34, 34 arranged opposite to each other, the electrodes 34, 34 are arranged opposite to each other in the first direction 40 in the first electropolishing step and the electrodes 34, 34 are arranged opposite to each other in the second direction 42 in the second electropolishing step. Therefore, the opposite sides of the metal pipe 10' in the second direction 42, where the amount of dissolution is smaller in the first electropolishing step, can be efficiently dissolved in the second electropolishing step. Thus, the differential in outer diameter dimension can be minimized between the two orthogonal directions where the differential in outer diameter dimension of the metal pipe 10' is likely to be large.

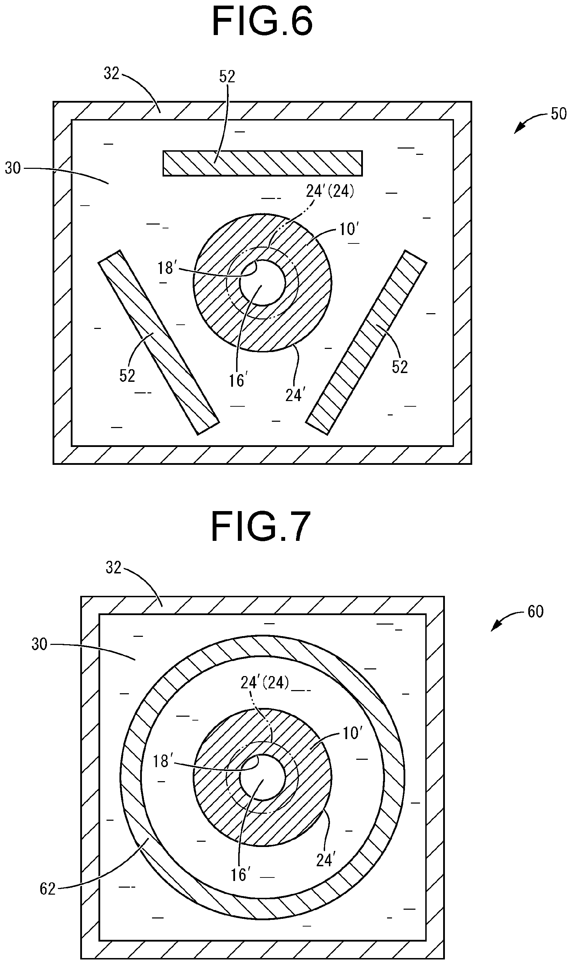

As for the electropolishing step in the manufacturing method of a conical hollow needle, the embodiment shown in FIGS. 5A and 5B is the most suitable among the embodiments disclosed in this description, but other embodiments shown in FIGS. 6 and 7 may also be employed. That is, FIG. 6 illustrates an electropolishing step in the manufacturing method of a conical hollow needle according to a second practical embodiment of the present invention. In the following description, components and parts that are substantially identical with those in the first practical embodiment will be assigned like symbols and not described in any detail. In addition, since the conical hollow needles manufactured by the second and third practical embodiments are substantially the same as the conical hollow needle 10 shown in the first practical embodiment, the explanation will be omitted.

In an electropolishing device 50 shown in FIG. 6, three electrodes 52, 52, 52 serving as cathodes are arranged around the metal pipe 10', which is the original form of the conical hollow needle. The three electrodes 52, 52, 52 incline at 120 degrees to one another, and are arranged to surround the metal pipe 10' at approximately equal intervals in the circumferential direction of the metal pipe 10'. Each electrode 52 extends in the tangential direction to the outer circumferential edge of the transverse cross section of the metal pipe 10', and extends approximately orthogonal to the radial direction of the metal pipe 10', which is the direction of opposition of the metal pipe 10' and the electrodes 52. The distance between each of three electrodes 52, 52, 52 and the opposite outer circumferential surface 20' of the metal pipe 10' is approximately constant.

In this way, the three electrodes 52, 52, 52 are arranged around the metal pipe 10', so that the three electrodes 52, 52, 52 are simultaneously arranged at three mutually different positions in the circumferential direction of the metal pipe 10', and are simultaneously opposite to the outer circumferential surface 20' of the metal pipe 10' at three positions. In electropolishing, all three electrodes 52, 52, 52 serve as the cathodes, and the metal pipe 10' serves as the anode. In the electropolishing device 50 of this practical embodiment, during the electropolishing step, the three electrodes 52, 52, 52 are fixedly arranged without moving relative to the outer circumferential surface 20' of the metal pipe 10', and the arrangement mode of the three electrodes 52, 52, 52 with respect to the outer circumferential surface 20' of the metal pipe 10' is not changed.

By passing an electric current between the metal pipe 10' and each electrode 52, the outer circumferential portion of the metal pipe 10' can be electrolyzed to reduce its diameter. Besides, as in the first practical embodiment, in the electropolishing step, by moving the metal pipe 10' and the electrolytic cell 32 relative to each other in the axial direction of the metal pipe 10' (the direction orthogonal to the paper surface in FIG. 6), the outer circumferential surface 24' of the electropolishing region 38 of the metal pipe 10' can be made into the tapered conical part 24.

According to this practical embodiment, the three electrodes 52, 52, 52 are simultaneously arranged at three positions in the circumferential direction with respect to the metal pipe 10'. This reduces variation in the efficiency of electropolishing in the circumferential direction of the metal pipe 10' without rotating the metal pipe 10' and the electrodes 52, 52, 52 relative to each other. Hence, a mechanism for rotating the metal pipe 10' and the electrodes 52, 52, 52 relative to each other is unnecessary, and a conical part with a transverse cross-sectional shape close to a perfect circle can be obtained by a simple manufacturing device.

In this practical embodiment, an example is shown in which three electrodes 52, 52, 52 are provided, but for example, four or more electrodes can be arranged simultaneously. For example, by arranging more electrodes, the roundness of the outer circumferential shape of the conical part can be improved. Each electrode 52 is not necessarily limited to a flat-plate shape, but can be curved or bent along the circumferential direction of the metal pipe 10', for example.

FIG. 7 illustrates an electropolishing step in the manufacturing method of a conical hollow needle according to a third practical embodiment of the present invention. In an electropolishing device 60 shown in FIG. 7, an electrode 62 serving as a cathode has a cylindrical shape that continuously surrounds the metal pipe 10', which is the original form of the conical hollow needle, over the entire circumference. The electrode 62 is arranged concentrically with respect to the metal pipe 10', and extends in a curved manner in the circumferential direction of the metal pipe 10', while being opposite to the outer circumferential surface 20' of the metal pipe 10' simultaneously over the entire circumference in the circumferential direction. It is desirable that the distance between the outer circumferential surface 20' of the metal pipe 10' and the opposite inner circumferential surface of the electrode 62 in the radial direction be approximately constant over the entire circumference.

According to this practical embodiment, since the metal pipe 10' and the electrode 62 are opposite to each other at an approximately constant distance over the entire circumference, the metal pipe 10' is electropolished approximately uniformly over the entire circumference. Therefore, in the conical part of the conical hollow needle, the circumferential variation in the outer diameter dimension is further reduced, thereby obtaining a conical part with a transverse cross-sectional shape close to a perfect circle by electropolishing.

Although it is also conceivable to use the electrolytic cell 32 itself as an electrode, it would difficult to set a sufficiently small distance between the electrode and the opposite metal pipe 10', and the shape of the electrolytic cell 32 is limited to a circular shape. This may cause troubles that it is difficult to realize an electrolytic cell 32 that can process a plurality of metal pipes 10', for example, and thus lacks practicality.

FIG. 8 illustrates an electropolishing step in the manufacturing method of a conical hollow needle according to a fourth practical embodiment of the present invention. This practical embodiment is based on the preceding first practical embodiment, and shows another embodiment of the plurality of electrodes 34 that are arranged apart from the outer circumference of the metal pipe 10' and the energization control for those plurality of electrodes 34. The vertical cross-sectional view is the mode in which, for example, as in the first practical embodiment, each electrode 34 is spaced apart on the outer peripheral side of the metal pipe 10' and extends approximately parallel to the metal pipe 10'.

Specifically, in this practical embodiment, the electrodes 34 serving as the cathodes, which are arranged apart on the outer peripheral side of the metal pipe 10', comprise two pairs of electrodes 34a, 34a and electrodes 34b, 34b, which are opposite to each other with the metal pipe 10' interposed therebetween in two diametrical directions orthogonal to each other. That is, a total of four electrodes 34 are arranged on the outer peripheral side of the metal pipe 10' so as to be separated from one another in the circumferential direction, and are arranged opposite to different positions of the outer circumferential surface of the metal pipe 10' in the circumferential direction. In this practical embodiment, a plurality of metal pipes 10' are arranged at prescribed intervals in an elongated electrolytic cell 32, so that the adjacent metal pipes 10', 10' can share the electrodes 34a, 34b arranged in the middle of them, and the total number of the electrodes 34a, 34b can be reduced in relation to the number of the metal pipes 10'.

In this way, two pairs of the electrodes 34a, 34a and the electrodes 34b, 34b serving as cathodes are arranged apart from and opposite to the circumference of the metal pipe 10'. In this embodiment, for example, regarding the one pair of electrodes 34a, 34a and the other pair of electrodes 34b, 34b, by controlling to alternately switch the energization state with respect to the metal pipe 10' by a timer switch or the like, the electropolishing region on the metal pipe 10' is sequentially switched between the surface region opposite to the one pair of electrodes 34a, 34a and the surface region opposite to the other pair of electrodes 34b, 34b. As a result, the outer circumferential surface of the metal pipe 10' is electropolished in each of the two mutually orthogonal directions, and compared to the case where, for example, electropolishing is performed simply by one pair of electrodes as in the preceding first practical embodiment, it is possible to easily perform electropolishing with a high degree of roundness on the metal pipe 10' without having to rotate the metal pipe 10' around the center axis with respect to the electrodes. Besides, in consideration of the difference in the amount of electropolishing in the circumferential direction of the metal pipe 10', by controlling energization time and applied voltage with respect to the metal pipe 10' to be different between the one pair of electrodes 34a, 34a and the other pair of electrodes 34b, 34b, it is also possible to improve the roundness of the metal pipe 10'.

FIG. 9 illustrates an electropolishing step in the manufacturing method of a conical hollow needle according to a fifth practical embodiment of the present invention. An electropolishing device 70 shown schematically in FIG. 9 has a structure in which an electrode device 72, which includes electrodes 74 serving as cathodes, is provided on each of the walls on opposite sides of the electrolytic cell 32 with the metal pipe 10' interposed therebetween. In the electrode device 72, a plurality of the electrodes 74 are arranged in the length direction of each wall of the electrolytic cell 32 with non-conductive spacer parts 76 interposed therebetween.

With this arrangement, the plurality of electrodes 74 in each electrode device 72 have different separation distances (the separation distances in the approximate direction of the metal pipe 10') from the surface of the metal pipe 10'. For example, the separation distance between the metal pipe 10' and the closest electrode 74 is indicated by Da in the drawing, and the separation distance between the metal pipe 10' and the farthest electrode 74 is indicated by Db in the drawing. Besides, the plurality of electrodes 74 are different from one another in the circumferential direction of the metal pipe 10' in terms of position of opposition to the surface of the metal pipe 10' in the direction of separation.

The energization between the plurality of electrodes 74 and the metal pipe 10' is performed by controlling voltage and/or current supplied by a power supply 78 for each electrode 74 with a controller 80. As a specific example, for each of the plurality of electrodes 74, the electrode with a smaller separation distance from the metal pipe 10' may be controlled such that the energization time is shorter or may be controlled such that the applied voltage is smaller than those of the electrode with a larger separation distance. This makes it possible to electropolish the outer circumferential surface of the metal pipe 10' with good roundness and time efficiency over the entire circumference.

FIG. 10 illustrates an electropolishing step in the manufacturing method of a conical hollow needle according to a sixth practical embodiment of the present invention. This practical embodiment is based on the preceding first practical embodiment, and shows another embodiment of FIG. 4C. The horizontal cross-sectional view can be, for example, the mode as shown in FIGS. 5A and 5B as in the first practical embodiment. That is, in this practical embodiment, an electrode 34 serving as a cathode, which is arranged apart on the outer peripheral side of the metal pipe 10', is divided in the depth direction of the electrolytic cell 32 into which the distal end of the metal pipe 10' is inserted (the length direction of the metal pipe 10'), so that a plurality of electrodes 34 are arranged in the depth direction of the electrolytic cell 32 with non-conductive spacer parts interposed therebetween. The plurality of electrodes 34 are different from one another in the length direction of the metal pipe 10' in terms of position of opposition to the surface of the metal pipe 10' in the direction of separation.

The energization between the plurality of electrodes 34 and the metal pipe 10' is performed by controlling voltage and/or current supplied by a power supply for each electrode 34 with a controller, similarly to the plurality of electrodes 74 in the preceding fifth practical embodiment. As a specific example, for each of the plurality of electrodes 34, the electrode closer to the upper opening of the electrolytic cell 32 may be controlled such that the energization time is shorter or may be controlled such that the applied voltage is smaller than those of the electrode closer to the bottom of the electrolytic cell 32. By so doing, the outer circumferential surface of the metal pipe 10' can be electropolished so as to become thinner toward the bottom. In such electropolishing, it is also possible to insert the entire length of the electropolishing region 24' of the metal pipe 10' into the electrolytic solution 30 from the beginning and perform electropolishing process.

While the present invention has been described in detail hereinabove in terms of the practical embodiments, the invention is not limited by the specific description thereof. For example, there may be only a single electrode 34. In the electropolishing step, by rotating the metal pipe 10' four times at 90 degrees per rotation, for example, the electrode 34 can be arranged at four positions in the circumferential direction with respect to the metal pipe 10'. When rotating the metal pipe 10' and the electrode 34 relative to each other, the amount of relative rotation of the metal pipe 10' and the electrode 34 is not limited. However, it is necessary to arrange the electrode 34 in not less than three positions in the circumferential direction with respect to the metal pipe 10' by the relative rotation, so that in preferred practice, the rotation angle is other than 180 degrees and multiples thereof. Whereas the first practical embodiment describes the case where the metal pipe is rotated stepwise by a prescribed angle with respect to the electrode, the metal pipe may alternatively be rotated continuously in the circumferential direction while performing electropolishing, for example.

The preceding first practical embodiment describes that in the electropolishing step, by gradually increasing the length of the metal pipe 10' immersed in the electrolytic solution 30, the immersion time in the electrolytic solution 30 is made longer at the distal end than at the proximal end. However, for example, from the state in which the entire electropolishing region 38 of the metal pipe 10' is immersed in the electrolytic solution 30, by gradually decreasing the length of the metal pipe 10' immersed in the electrolytic solution 30, the immersion time in the electrolytic solution 30 can be made longer at the distal end than at the proximal end. The process of gradually increasing the length of the metal pipe 10' immersed in the electrolytic solution 30 and the process of gradually decreasing the length of the metal pipe 10' immersed in the electrolytic solution 30 may be performed sequentially, or they may be repeated alternately.

By changing the distance between the metal pipe 10' and the opposite electrode 34 so as to increase from the distal end to the proximal end of the metal pipe 10', for example, it is also possible to change the amount of dissolution in the axial direction with the same process time of electropolishing.

The preceding practical embodiment shows an example in which the immersion length of the metal pipe 10' in the electrolytic solution 30 is changed by moving the metal pipe 10' and the electrolytic cell 32 relative to each other in the axial direction of the metal pipe 10'. Alternatively, for example, by raising or lowering the level of the electrolytic solution 30 in the electrolytic cell 32, the immersion length of the metal pipe 10' in the electrolytic solution 30 can be changed without moving the metal pipe 10' and the electrolytic cell 32 relative to each other in the axial direction of the metal pipe 10'.

It would be acceptable as long as the conical part 24, which is the electropolished surface, constitutes at least a part of the outer circumferential surface 20 of the conical hollow needle 10. The entire outer circumferential surface 20 may be constituted by the conical part 24, or only the distal end portion of the outer circumferential surface 20 may be constituted by the conical part 24 as in the preceding practical embodiment.

Regarding the outer circumferential surface 20 of the conical hollow needle 10, it is not absolutely necessary that only the conical part 24 serves as the electropolished surface. For example, the cylinder part 22 can also be made smooth by electropolishing. Therefore, the electropolishing region 38 of the metal pipe 10' is not necessarily limited to only the region where the conical part 24 is formed.

In the preceding practical embodiment, for ease of understanding, the case where a single metal pipe 10' is electropolished is described, but it is also possible, for example, to electropolish a plurality of metal pipes 10' simultaneously. In this case, the plurality of metal pipes 10' may be electropolished simultaneously in a single electrolytic cell 32, or the plurality of metal pipes 10' may be electropolished simultaneously in a plurality of respective electrolytic cells 32. For example, two elongated electrodes 34, 34 are arranged opposite to each other at a prescribed distance in a single electrolytic cell 32, and a plurality of metal pipes 10' are disposed between the said two electrodes 34, 34, which are arranged with opposite faces parallel to each other at a prescribed distance, side by side at a prescribed distance in the length direction of the electrodes 34. This makes it possible to electropolish the plurality of metal pipes 10' simultaneously between the two electrodes 34, 34. Then, by rotating the plurality of pipes 10' at prescribed time intervals or continuously around their respective central axes, it is possible to make the direction of opposition of the electrode 34 with respect to each metal pipe 10' to be different in four or more directions while the two electrodes 34, 34 are fixedly located. By so doing, a plurality of needles with a high degree of roundness can be efficiently manufactured.

In the method of the present invention, the direction of opposition of the cathode with respect to the outer circumferential surface of the hollow needle means the direction of opposition in which the distance between the hollow needle and the cathode is shortest. Therefore, if the cathode has a flat-plate shape, it only provides a single direction of opposition even if the cathode extends in the circumferential direction of the hollow needle. On the other hand, if the cathode has a curved shape with a curved opposite face whose center of curvature is the central axis of the hollow needle, even if there is only a single cathode, it can provide directions of opposition at not less than three different positions in the circumferential direction of the hollow needle, similarly to the cylindrical cathode surrounding the hollow needle shown in the preceding third practical embodiment. Indeed, in the present invention, the cathode, which realizes the directions of opposition in the radial direction of the hollow needle at not less than three different positions, preferably provides not less than three directions of opposition at equal intervals (at equal angles) in the circumferential direction of the hollow needle evenly in time during the electropolishing process.

Although the preceding practical embodiment describes the conical hollow needle 10 having the needle tip 14 only at one end, the present invention can be applied, for example, to a double-ended needle having needle tips at the opposite ends. In this case, for example, the conical part 24 may be formed only at the distal end portion, which is the side to puncture the patient, or the conical parts 24 may be formed at the opposite end portions. Besides, in the conical hollow needle 10 having the needle tip 14 only at the distal end, a needle hub or the like may be provided at the proximal end.

### KEYS TO SYMBOLS

- 10: conical hollow needle (first practical embodiment)
- 10': metal pipe (hollow needle)
- 12: blade surface
- 14: needle tip
- 16: lumen (lumen of conical hollow needle)
- 16': lumen (lumen of hollow needle)
- 18: inner circumferential surface (inner circumferential surface of conical hollow needle)
- 18': inner circumferential surface (inner circumferential surface of hollow needle)
- 20: outer circumferential surface (outer circumferential surface of conical hollow needle)
- 20': outer circumferential surface (outer circumferential surface of hollow needle)
- 22: cylinder part
- 24: conical part
- 24': outer circumferential surface (outer circumferential surface of electropolishing region)
- 26: electropolishing device
- 28: anode side wiring
- 30: electrolytic solution
- 32: electrolytic cell
- 34: electrode (cathode)
- 36: cathode side wiring
- 38: electropolishing region
- 40: first direction
- 42: second direction
- 50: electropolishing device (second practical embodiment)
- 52: electrode (cathode)
- 60: electropolishing device (third practical embodiment)
- 62: electrode (cathode)
- 70: electropolishing device (fifth practical embodiment)
- 72: electrode device
- 74: electrode (cathode)
- 76: spacer part
- 78: power supply
- 80: controller

## Claims

1. A method of manufacturing a conical hollow needle including a conical part whose outer circumferential surface is tapered, comprising
immersing a hollow needle having a straight shape in an electrolytic solution and performing electropolishing to make an outer circumferential surface of the hollow needle into a conical shape, wherein
from a start to an end of the electropolishing, a pair of cathodes are arranged such that the cathodes sandwich the hollow needle serving as an anode at a prescribed distance in a radial direction, and the hollow needle is rotated around a needle axis to change which faces of the hollow needle are opposite to the cathodes.

2. A method of manufacturing a conical hollow needle including a conical part whose outer circumferential surface is tapered, comprising
immersing a hollow needle having a straight shape in an electrolytic solution and performing electropolishing to make an outer circumferential surface of the hollow needle into a conical shape, wherein
from a start to an end of the electropolishing, at least one cathode is arranged radially opposite to the outer circumferential surface of the hollow needle serving as an anode, the at least one cathode being arranged at not less than three different positions in a circumferential direction of the hollow needle.

3. The method according to claim 2, wherein
the at least one cathode comprises a plurality of cathodes arranged independently of each other in the circumferential direction of the hollow needle, and
from the start to the end of the electropolishing, an arrangement of the plurality of cathodes relative to the hollow needle is changed so that the plurality of cathodes are arranged at different positions in the circumferential direction of the hollow needle.

4. The method according to claim 2, wherein
the at least one cathode is arranged to surround the hollow needle, and
from the start to the end of the electropolishing, the at least one cathode is simultaneously opposite to the hollow needle at not less than three different positions in the circumferential direction.

5. The method according to any one of claims 2-4, wherein the at least one cathode extends in a curved manner in the circumferential direction of the hollow needle.

6. The method according to any one of claims 1-5, wherein the conical part with a roundness of 0 to 0.0015 mm is formed by the electropolishing.

7. A method of manufacturing a conical hollow needle including a conical part whose outer circumferential surface is tapered, comprising
immersing a hollow needle having a straight shape in an electrolytic solution and performing electropolishing to make an outer circumferential surface of the hollow needle into a conical shape, wherein
a plurality of cathodes are arranged apart from and opposite to the outer circumferential surface of the hollow needle serving as an anode, and the electropolishing on the hollow needle by each cathode is adjusted by controlling an energization state between the hollow needle and the plurality of cathodes.
